# EUROPÄISCHE PATENTANMELDUNG

(11) **EP 0 622 057 A2**
(43) Veröffentlichungstag der Anmeldung: **02.11.1994**
(21) Anmeldenummer: 94850067.3
(22) Anmeldetag: 26.04.1994
(51) Int. Cl.: A61F 2/00, A61C 8/00, A61F 11/04

(54) **Zur Implantation im Knochengewebe bestimmte Haltevorrichtung zur kontrollierten Aufnahme und Lagefixierung von vorzugsweise zur elektrischen Informationsüberführung verwendbarer Ausrüstung**

(30) Priorität: 27.04.1993 SE 9301406
(71) Anmelder: MEDEVELOP AKTIEBOLAG, S-402 29 Göteborg (SE)
(72) Erfinder: Branemark, Per-Ingvar, S-431 69 Mölndal (SE)
(74) Vertreter: Karlsson, Leif Karl Gunnar

(57) **Zusammenfassung**

Eine mit Knochengeweben integrierbare, vorzugsweise zur elektrischen Überführung von Information zum Innenohr vorgesehene, Haltevorrichtung (1) mit einem Einführungsende (2) zur Einführung der Haltevorrichtung in eine vorpräparierte Bohrung im Knochengewebe, einem äusseren Anschlussende (3), einer durchgehenden, zentrierten Bohrung (4), die die beiden Enden (2, 3) des Körpers verbindet, einem am Einführungsende vorgesehenen Aussengewinde (7) und einem in der Bohrung vorgesehenen, mit einem elektrischen Leiter (8) versehenen Kontaktelement (9) auf. Der Leiter (8) geht vom Kontaktelement aus und erstreckt sich durch die Bohrung am Einführungsende und/oder Öffnungen in der Manteloberfläche des Körpers (1).

## Beschreibung

Die vorliegende Erfindung betrifft eine zur Implantation im Knochengewebe bestimmte, rotationssymmetrisch ausgebildete und aus gewebekompatiblem Material bestehende Haltevorrichtung zur kontrollierbaren Aufnahme und Lagefixierung von zur elektrischen Informationsüberführung verwendbarer Ausrüstung, wobei die Haltevorrichtung ein von ihrem einen Ende (Anschluss) ausgehendes und sich zum entgegengesetzten Ende (Einführungsende) erstreckendes Aussengewinde aufweist.

Rotationssymmetrisch ausgebildete, zur Implantation im Gewebe bestimmte und mit einem Aussengewinde versehene Verankerungsvorrichtungen aus gewebeverträglichem Material sind beispielsweise in US-A- 5 064 425 beschrieben. Solche Verankerungsvorrichtungen wurden mit grossem Erfolg zur Halterung künstlicher Zähne, Zahnbrücken und auch Prothesen, künstlicher Gelenke bei Rekonstruktion von Gelenken u.s.w verwendet. Bezüglich der dentalen Verwendung werden derartige Verankerungsvorrichtungen von NobelPharma AB, Schweden, under dem Warenzeichen BRÅNEMARK SYSTEM® auf dem Markt vertrieben.

Seit langem liegt ein Bedürfnis für einen kombinierten Hautdurchgang und einer kontrollierbaren Lagefixierung für andere Ausrüstung vor, beispielsweise zwecks Informationsüberführung zum Innenohr bei schwerhörigen Personen.

Gegenstand vorliegender Erfindung ist die Schaffung eines derartigen Halters, der teils in kontrollierbarer Weise durch Verankerung im Knochengewebe lagefixiert werden kann und teils eine einstellbare Aufnahme dafür benötigter Anschlüsse ermöglicht.

Bei der gattungsgemäss beschriebenen Haltevorrichtung wird diese Aufgabe hauptsächlich durch eine vom Einführungsende ausgehende Innenbohrung erzielt, die sich bis zum Anschlussende der Haltevorrichtung erstreckt und/oder mit einer oder mehreren in der gewindeversehenen Manteloberfläche der Haltevorrichtung vorgesehenen Öffnungen in Verbindung steht, wobei die Innenbohrung einen Durchgang zur Aufnahme von Anschlüssen zu der genannten Ausrüstung bildet.

Nach einer geeigneten Ausführungsform der Erfindung ist das Anschlussende der Haltevorrichtung zur Aufnahme von mit den genannten Anschlüssen zusammenwirkenden Kontaktelementen und Kontaktorganen versehen.

Die Haltevorrichtung ist zweckmässig mit einer Innenbohrung mit verschiedenen Innendurchmessern versehen, wobei der den grössten Durchmesser aufweisende Abschnitt der Innenbohrung am Anschlussende der Bohrung angeordnet ist.

Zweckmässig ist die Haltevorrichtung am Anschlussende mit Verriegelungsorganen zur Lagefixierung des Kontaktelements.

Hierbei weist das Kontaktelement zweckmässig Steckkontaktorgane oder Kontaktbuchsenorgane zum leitenden Anschluss des Stromleiters an ein oder mehrere ausserhalb der Haltevorrichtung vorgesehene Aggregate auf, wie beispielsweise signalerzeugende, signalempfangende Aggregate und Stromversorgungsaggregate.

Nach einer zweckmässigen Ausführungsform ist das Einführungsende der Haltevorrichtung mit selbständig gewindeschneidenden Schlitzen versehen, die sich vom Einführungsende der Haltevorrichtung in Richtung zum Anschlussende erstrecken und wobei die Längskanten der Schlitze als Schneidkanten ausgebildet sind.

Nach einer weiteren Ausführungsform der Erfindung umfasst die Haltevorrichtung einen ersten Halterabschnitt mit einem erstem Aussengewinde und einer ersten Zentralbohrung sowie einen an den ersten Halterabschnitt einstückig axiell angeschlossenen, zylindrischen zweiten Halterabschnitt, der eine zweite Zentralbohrung aufweist, wobei der erste und der zweite Halterabschnitt und die Zentralbohrung rotationssymmetrisch um eine Rotationsachse angeordnet sind und der erste Halterabschnitt mit seinem freien Ende in eine im Knochengewebe vorpräparierte Bohrung einführbar ist und der Aussendurchmesser des zweiten Halterabschnitts wenigstens gleich gross wie der Aussendurchmesser des ersten Halterabschnitts ist, wobei darüber hinaus der Durchmesser der zweiten Zentralbohrung grösser ist als der Durchmesser der ersten Zentralbohrung sowie die zweite Zentralbohrung zum Tragen des Kontaktelements und die erste Zentralbohrung zur Aufnahme des vom Kontaktelement ausgehenden elektrischen Leiters ausgebildet ist.

Hierbei ist die Haltevorrichtung wenigstens in ihren Teilen, die nach der Implantation Gewebekontakt haben, aus Titan oder einem anderen gewebeverträglichen Material und ist wenigstens im Bereich ihrer Oberfläche, der nach der Implantation Gewebekontakt hat, mit einer mit Mikrodellen versehenen Oberfläche versehen, wobei der Durchmesser der Dellen zwischen 10 und 1.000 nm beträgt, vorzugsweise zwischen 10 und 300 nm.

Hierdurch werden Voraussetzungen für eine optimale Verankerung bzw Osseointegration der Zellenausläufer der Gewebezellen in bzw. mit den genannten Mikrodellen geschaffen.

Die Erfindung wird nachstehend anhand einiger in beigefügten Zeichnungen gezeigten Ausführungsformen näher beschrieben. Es zeigen:
- Fig. 1: eine erste Ausführungsform der erfindungsgemässen Haltevorrichtung im Längsschnitt durch die Rotationsachse R,
- Fig. 2: eine Variante der Haltevorrichtung nach Fig. 1 zeigt, in gleicher Ansicht und mit einem angeschlossenen Steckkontakt,
- Fig. 3: eine zweite Ausführungsform der Haltevorrichtung nach Fig. 1 im Längsschnitt durch die Rotationsachse R' und
- Fig. 4: eine dritte Ausführungsform der erfindungsgemässen Haltevorrichtung im Längsschnitt durch die Rotationsachse R''.

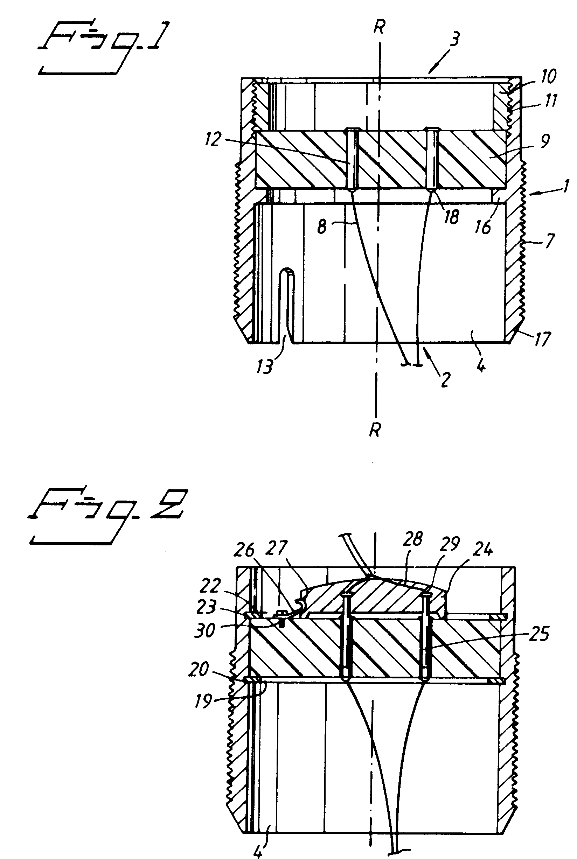

Die in Fig. 1 gezeigte Haltevorichtung umfasst grundsätzlich einen zylindermantelförmigen Körper 1 aus Titan, der mit einer Innenbohrung versehen ist, und ein sich vom einen Ende 2 (Einführungsende) in Richtung auf das entgegengesetzte Ende 3 (Anschlussende) erstreckendes Aussengewinde 7 aufweist. In einem Abschnitt am Anschlussende 3 ist die Mantelaussenfläche gewindelos.

In der hier gezeigten Ausführung ragt jedoch ein innerer Ringflansch 16 von der Innenbohrung 4 etwa in der Mitte zwischen den Enden 2, 3 hervor, auf dessen dem Anschlussende zugekehrter Fläche ein zylinderförmiges Kontaktelement 9 aus Polypropenplast ruht; das Kontaktelement 9 liegt hierbei dicht gegen die Wand der Bohrung 4. Ein am Anschlussende 3 naheliegender Abschnitt der Wand der Bohrung 4 ist mit einem Innengewinde 11 zur Aufnahme einer mit einem entsprechenden Aussengewinde versehener Stoppschraube 10 versehen, wodurch das Kontaktelement 9 in seiner Lage gegen den Ringflansch 16 fixiert wird.

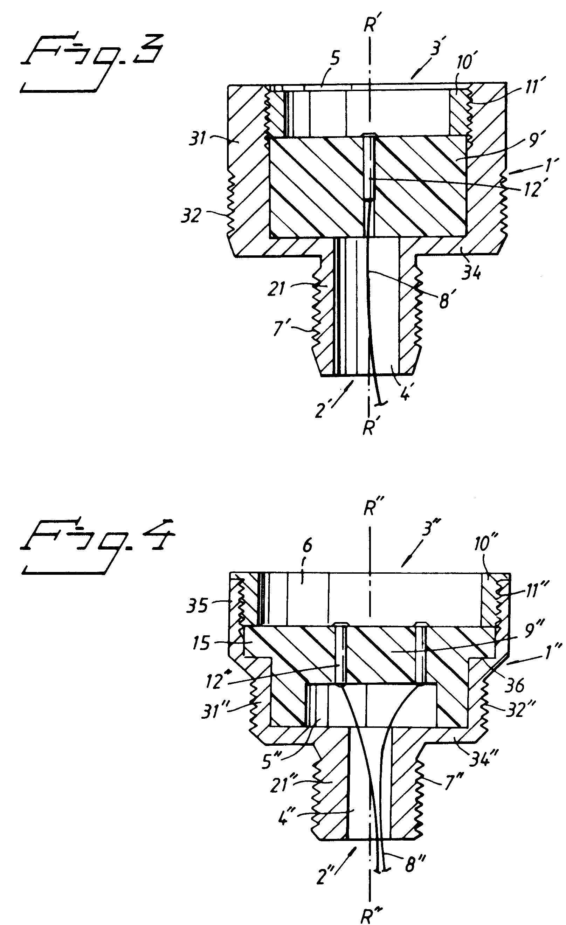

Am Einführungsende 2 sind in Längsrichtung drei in Richtung zum Anschlussende 3 verlaufende Schlitze oder Einschnitte 13 im Zylindermantel des Körpers 1 angebracht; nur ein solcher Schlitz 13 ist in Fig. 2 gezeigt. Diese Schlitze 13 sind mit Schneidkanten versehen, wodurch ein selbstschneidender Effekt beim Einschrauben der Haltevorrichtung 1 in eine im Knochengewebe vorpräparierte Bohrung erreicht wird. Die Wandstärke des Körpers 1 wird in Richtung zum Einführungsende in einem von diesem Ende 2 ausgehenden Wandabschnitt 17 keilförmig schmäler (ist abgeschrägt).

Darüber hinaus erleichtert diese Endabschrägung das Anbringen des Einführungsendes 2 der Haltevorrichtung gegen oder, genauer gesagt, in der im Knochen angebrachten Bohrung und das Eingreifen des selbstschneidenden Gewindes 7 in den Wänden der Bohrung.

Das Kontaktelement 9 ist mit zwei axiell orientierten, durchgehenden Kontaktbuchsen 12 mit Wänden aus elektrisch leitendem Material versehen, an welchen eine dünne, biegsame Leitung 8, beispielsweise in Form einer Kupferlegierung, mittels Lötung am inneren Kontaktende 18 angeschlossen ist. Die Leitung 8 ist isoliert (wird hier in Fig. 1 nicht besonders angedeutet) und verlässt die Bohrung am Einführungsende 2. Gemäss einer in der Zeichnung nicht gezeigten alternativen Ausführung können entlang der Manteloberfläche des Körpers 1 ausserdem eine oder mehrere Seitenöffnungen vorgesehen sein, die in diesem Fall auch einen Seitendurchgang der Leitung 8 zulassen. In die Kontaktbuchsen 12 kann vom äusseren Ende 3 her ein passender Steckkontakt eingeführt und gekuppelt werden, vgl Fig. 2.

Bei der in Fig. 2 gezeigten Variante der in Fig. 1 gezeigten Ausführungsform ist der ringförmige Absatz 16 durch einen federnden inneren Sicherungsring 19 ersetzt, der in einer in die Innenwand der Bohrung 4 eingefrästen Nut 20 befestigt ist. In entsprechender Weise ist der Sicherungsring 10 mit zugehörigem Innengewinde 11 durch einen federnden äusseren Sicherungsring 22 ersetzt, der in einer zur Achse R senkrechten Nut 23 auf der Innenseite des Körpers 1 zusammenwirkt. Das Kontaktelement 9 wird zwischen den Sicherungsringen 19 und 22 festgehalten. In Fig. 2 wird auch ein zu dem fest angebrachten Kupplungselement 9 passendes lösbares Kupplungsorgan 24 mit einer isolierten Zuleitung 28 gezeigt, die bei 29 an einen Steckkontakt 25 angelötet ist. Das Kupplungsorgan 24 wird in mit dem fest angebrachten Kupplungselement 9 gekuppelten Zustand gezeigt. Das Kupplungselement 9 und das Kupplungsorgan 24 werden in ihrer Lage zueinander u.a. mit einer auf dem fest montierten Kupplungselement 9 mittels einer Schraube 30 befestigten Blattfeder 26 fixiert, wobei letztere in eine Aussparung 27 an der Seitenwand des Kupplungsorgans 24 eingreift. Die Variante nach Fig. 2 erlaubt eine einstufige Fertigung der Innenbohrung 4, erfordert jedoch andererseits ein Ausfräsen der Nuten 20 und 23. Diese Ausführung hat den Vorteil, dass ein Körper 1 bestimmter Form zur Aufnahme von Kupplungselementen 9 verschiedener Dicke angepasst werden kann und bietet auch einen grösseren Spielraum bezüglich des Anordnens des Kupplungselementes 9 in der Bohrung 4.

Die in Fig. 3 gezeigte Ausführung hat die Form von zwei an ihren Wandungen einstückig miteinander verbundener Zylindermäntel, deren Zylinderachsen sich decken, hier als erste und zweite Halterelemente 21, 31 bezeichnet; der erste Zylinder geht hierbei von der Wandung 34 des zweiten Zylinders aus. Die durchgehende Innenbohrung weist zwei Abschnitte auf, eine erste Zentralbohrung 41 im ersten Verankerungselement 21 mit einem freien, als Einführungsende bezeichnetem Teil 2', und einer zweiten Zentralbohrung 5 im zweiten Verankerungselement 31 mit einem freien, als Anschlussende bezeichneten Teil 3'. Der Aussendurchmesser des ersten Halterteils 21 ist bedeutend kleiner als der Durchmesser der Bohrung 5 des zweiten Halterteils 31. Das zylinderförmige Kontaktelement 9' aus isolierendem Polymermaterial sitzt auf dem noch vorhandenen Abschnitt 34 des zweiten Halterteils 31 auf. Das Kontaktelement weist nur eine Kontakthülse 12' auf. Der zur Kontakthülse mit isolierter Leitung 8' gehörende kupplungsbare Stiftkontakt ist in Fig. 3 nicht gezeigt. In gleicher Weise wie bei der Haltevorrichtung in Fig. 1 ist das Kontaktelement 9' in der Bohrung mit Hilfe einer Stoppschraube 10' mit Aussengewinde fixiert, die in einem in der Bohrung 5 des zweiten Halterteils 31 angebrachten Innengewinde 11' eingeschraubt ist. Sowohl das erste als auch das zweite Halterteil 21, 31 haben Aussengewinde 7, 32, mit gleicher Gangsteigung, aber unterschiedlichen Aussendurchmessern. Das Aussengewinde 32 erstreckt sich jedoch nur über die Hälfte der Aussenwand des zweiten Halterteils, so dass die am äusseren Ende 3' gelegene Hälfte der Mantelfläche gewindelos ist.

In Fig. 4 wird eine weitere Ausführungsform der erfindungsgemässen Haltevorrichtung veranschaulicht, die in ihrem prinzipiellen Aufbau mit der in Fig. 3 gezeigten Ausführungsform übereinstimmt, d.h. ein erstes Halterteil 21'', ein zweites Halterteil 31'', einen mit beiden Teilen einstückig verbundenen Abschnitt 34'', eine durchgehende Innenbohrung mit einem ersten Abschnitt 4'' im ersten Halterteil und einem zweiten Abschnittt 5'' im zweiten Halterteil 31'', ein freies Einführungsende 2'' am ersten Halterteil 21'' und ein erstes und ein zweites Aussengewinde 7'', 32'' aufweist. Diese Teile verhalten sich zueinander in gleicher Weise wie die entsprechenden Teile der in Fig. 3 gezeigten Ausführungsform, ausgenommen davon, dass sich das zweite Aussengewinde 32'' über fast die gesamte Manteloberfläche des zweiten Halterteils 31'' erstreckt. Das zweite Halterteil 31'' ist jedoch an einem vom ersten Halterteil 21 abgekehrten Ende einstückig mit einem zylindermantelförmigen, in gleicher Weise wie das Halterteil zentrierten Flanschelement 35 verbunden, dessen Aussenradius und Innenradius grösser sind als der Aussenradius bzw. Innenradius des zweiten Halterteils 31''. Die Verbindung zwischen dem Flanschelement 35 und dem zweiten Halterteil 31'' bildet einen Absatz 36, gegen den eine in gleicher Weise wie in den vorhergehenden Beispielen ausgebildete, in ein Innengewinde 11'' am Flanschelement 35 eingeschraubte Stoppschraube 10'' einen radiellen Flanschabschnitt 15 des Kontaktelements 9'' fixiert; der Flansch 15 hat eine gute Passform im Flanschelement 35. Mit Ausnahme des genannten Flanschabschnitts 15 und einer zentralen Aussparung auf der dem Einführungsende 2'' zugekehrten Seite des Kontaktkörpers 9'' ist der Kontaktkörper 9'' in gleicher Weise wie in dem in Fig. 1 und 2 gezeigten Beispiel ausgebildet.

Selbstverständlich können die in Fig. 3 und 4 gezeigten Ausführungsformen, falls gewünscht, mit geraden oder spiralförmigen Schlitzen, abgeschrägten Einführungsendabschnitten, Stopp- und Sicherheitsringen u.s.w. versehen werden, in ähnlicher Weise wie bei den in Fig. 1 und 2 gezeigten Ausführungsformen.

Beim Einoperieren der in Fig. 1 und 2 gezeigten Haltevorrichtung wird der Knochen freipräpariert und im Knochen eine Bohrung angebracht, wobei die Tiefe der Bohrung der beabsichtigten Einführungstiefe und der Durchmesser hauptsächlich dem Innendurchmesser des Aussengewindes 7 entspricht. In Abhängigkeit von einem etwaigen Durchdringen des Knochens vom Halter kann es notwendig sein, die erste Bohrung zu verlängern, bis sie den Knochen durchdringt, oder in deren Boden eine Bohrung geringeren Durchmessers anzubringen zwecks einer Verlegung der Leitung 8. Sowohl die Verlegung der Leitung 8 wie das Anordnen einer besonderen Bohrung kann sowohl vor als auch nach dem Einschrauben der Haltevorrichtung vorgenommen werden. Falls die Leitung 8 später über das freie Ende 3 eingeführt wird, kann sie schon mit dem Kontaktelement 9 beispielsweise durch Löten fest verbunden sein. Nach Einschieben in die Bohrung 4 an dessen freiem Ende 3 und Absicherung in der Bohrung mittels Sicherungsring 10 oder Federring 23 ist die Vorrichtung komplett. Die Einschraubtiefe wird so gewählt, dass der gewindelose Bereich des Haltermantels sich von der Knochenhaut nach aussen hin erstreckt. Eventuell erhält die Manteloberfläche lokal eine spezielle Form und/oder Oberflächenbehandlung, um einen guten Hautdurchgang sicherzustellen, falls ein solcher aktuell ist, beispielsweise bei einer Kochlearprothese.

Die in Fig. 3 und 4 gezeigten Halterteile werden in ähnlicher Weise einoperiert, jedoch mit dem Unterschied, dass zwei sich in ihrer Achse deckende Bohrungen im Knochen angebracht werden, wobei die erste einen grösseren, dem Innendurchmesser des Aussengewindes 32, 32'' entsprechenden Durchmesser, und die zweite einen kleineren, dem Innendurchmesser des Aussengewindes 7, 7'' entsprechenden Durchmesser hat, und mit einer Mindesttiefe entsprechend der axiellen Länge des ersten Halterteils 21, 21''.

Die erfindungsgemäss vorgeschlagene Haltevorrichtung kann vorteilhaft zur Informationsüberführung ins Innenohr, aber auch für andere Zwecke, verwendet werden. In oder am Halter können auch Mikrochips, Batterien und andere elektrische Ausrüstung angeordnet werden. Das Kontaktelement kann auch mit einer verschliessbaren Öffnung versehen werden, beispielsweise einer Membran, zum Zwecke der Injektion einer Lösung, beispielsweise einer Antibiotikalösung, um Infektionen vorzubeugen oder zu bekämpfen.

## Patentansprüche

1. Zur Implantation im Knochengewebe bestimmte, rotationsymmetrisch ausgebildete und aus gewebeverträglichem Material bestehende Haltevorrichtung (1, 1'', 1'') zur steuerbaren Aufnahme und Lagefixierung von vorzugsweise zur elektrischen Informationsüberführung verwendbarer Ausrüstung, wobei die Haltevorrichtung ein von ihrem Einführungsende (2, 2', 2'') ausgehendes und sich in Richtung zum entgegengesetzten Anschlussende erstreckendes Aussengewinde (7, 7',7'') aufweist, **gekennzeichnet** durch eine vom Anschlussende (3, 3', 3'') der Haltevorrichtung (1, 1', 1'') ausgehende Innenbohrung (4, 4', 4''), die sich bis zum Einführungsende (2, 2', 2'') der Haltevorrichtung erstreckt und/oder mit einer oder mehreren in der mit Gewinde versehenen Manteloberfläche der Haltevorrichtung vorgesehenen Öffnungen in Verbindung steht, wobei die Innenbohrung (4, 4', 4'') einen Durchgang zur Aufnahme von Anschlüssen (8, 9, 12, 24) der genannten Ausrüstung bildet.

2. Haltevorrichtung nach Anspruch 1, dadurch **gekennzeichnet,** dass das Anschlussende (3, 3', 3'') der Haltevorrichtung (1, 1′, 1˝) zur Aufnahme von mit den genannten Anschlüssen zusammenwirkenden Kontaktelementen (9) und Kontaktorganen (24) vorgesehen ist.

3. Haltevorrichtung nach Anspruch 2, dadurch **gekennzeichnet,** dass die Innenbohrung Abschnitte mit verschiedenen Innendurchmessern (4′, 5; 4˝, 5'', 6) aufweist.

4. Haltevorrichtung nach Anspruch 3, dadurch **gekennzeichnet,** dass der Abschnitt der Innenbohrung mit dem grössten Durchmesser (5; 6) in der Nähe des Anschlussendes (3, 3', 3'') der Bohrung angeordnet ist.

5. Haltevorrichtung nach einem der Ansprüche 1 - 4, dadurch **gekennzeichnet** dass die Haltevorrichtung an ihrem Anschlussende (3, 3', 3'') mit Verriegelungsorganen (10, 11; 10′, 11′; 10˝, 11˝) zur Lagefixierung des Kontaktelements versehen ist.

6. Haltevorrichtung nach einem der Ansprüche 1 - 5, dadurch **gekennzeichnet,** dass das Kontaktelement Steckkontaktorgane oder Kontaktbuchsenorgane (12; 12'; 12'') zum leitenden Anschluss des Stromleiters an ein oder mehrere ausserhalb der Haltevorrichtung vorgesehene Aggregate, wie beispielsweise signalerzeugende, signalempfangende Aggregate und Stromversorgungsaggregate, aufweist.

7. Haltevorrichtung nach einem der Ansprüche 1 - 6, dadurch **gekennzeichnet,** dass vom Einführungsende (2, 2', 2'') der Haltevorrichtung ein oder mehrere axielle Schlitze (13) sich in Richtung zum Anschlussende (3, 3', 3'') erstrecken, wobei die Längskanten der Schlitze (13) als Schneidkanten ausgebildet sind.

8. Haltevorrichtung nach einem oder mehreren der vorhergehenden Ansprüche, **gekennzeichnet** durch einen ersten, hauptsächlich zylinderförmigen Halterabschnitt (21) mit einem erstem Aussengewinde (7') und einer ersten Zentralbohrung (4′) sowie einen an den ersten Halterabschnitt (21) einstückig axiell angeschlossenen, zylindrischen zweiten Halterabschnitt (31) mit einer zweiten Zentralbohrung (5), wobei der erste und der zweite Halterabschnitt (21, 31) und die Zentralbohrung (4′; 5) rotationssymmetrisch in bezug auf eine Rotationsachse (R′) angeordnet sind, wobei der erste Halterabschnitt (21) mit seinem freien Ende (2') in eine im Knochengewebe vorpräparierte Bohrung einführbar ist, der Aussendurchmesser des zweiten Halterabschnitts (31) wenigstens gleich gross wie der Aussendurchmesser des ersten Halterabschnitts (21) ist und der Durchmesser der zweiten Zentralbohrung (5) grösser ist als der Durchmesser der ersten Zentralbohrung (5) sowie die zweite Zentralbohrung zum Tragen des Kontaktelements (9') und die erste Zentralbohrung (4') zur Aufnahme des vom Kontaktelement ausgehenden elektrischen Leiters (8') ausgebildet ist.

9. Haltevorrichtung nach Anspruch 8, dadurch **gekennzeichnet,** dass der zweite Halterabschnitt (31) mit einem zweiten Aussengewinde (32) versehen ist, das die gleiche Steigung wie das erste Aussengewinde (7') aufweist.

10. Haltevorrichtung nach Anspruch 8 oder 9, dadurch **gekennzeichnet,** dass der zweite Halterabschnitt (31) mit Verriegelungsorganen zur Lagefixierung des Kontaktelements versehen ist, wobei die Verriegelungsorgane vorzugsweise ein an der zweiten Zentralbohrung, vorzugsweise an deren offenem Ende, angeordnetes Innengewinde (11') und einen mit Aussengewinde versehenen Sicherungsring (10') umfassen.

11. Haltevorrichtung nach Anspruch 8 - 10, dadurch **gekennzeichnet,** dass die Haltevorrichtung ein Flanschelement (35) umfasst, wobei der zweite Halterabschnitt (31'') an seinem vom ersten Halterabschnitt (4'') abgekehrten Ende einstückig an das in bezug auf die Rotationsachse (R'') rotationssymmetrisch angeordnete, an seinem offenen Ende (3'') mit Innengewinde versehenen Flanschelement (35) angeschlossen ist und dass der Durchmesser der Innenbohrung (6) des Flanschelements (35) grösser ist als der Durchmesser der Innenbohrung des zweiten Halterabschnitts (5'').

12. Haltevorrichtung nach Anspruch 11, dadurch **gekennzeichnet,** dass das Kontaktelement (9˝) an seinem Ende einen Ringflansch (15) aufweist, wobei der Aussendurchmesser des Ringflansches (15) im wesentlichen mit dem Durchmesser der Innenbohrung (6) des Flanschelements (35) übereinstimmt.

13. Haltevorrichtung nach einem der Ansprüche 7 - 12, dadurch **gekennzeichnet,** dass der vom Einführungsende ausgehende Schlitz (13) spiralförmig ist und die gleiche Chiralität wie das erste Aussengewinde (7; 7'; 7'') aufweist.

14. Haltevorrichtung nach einem oder mehreren der vorhergehenden Ansprüche, dadurch **gekennzeichnet,** dass die Haltevorrichtung wenigstens in ihren Teilen, die nach der Implantation Gewebekontakt haben, aus Titan oder einem anderen gewebeverträglichen Material besteht und wenigstens auf dem Teil ihrer Oberfläche, der nach der Implantation Gewebekontakt hat, eine mit Mikrodellen versehene Oberfläche aufweist, wobei der Durchmesser der Dellen zwischen 10 und 1.000 nm, vorzugsweise zwischen 10 und 300 nm, beträgt.
